# EUROPEAN PATENT APPLICATION

(11) **EP 1 095 650 A1**
(43) Date of publication of application: **02.05.2001**
(21) Application number: 99203578.2
(22) Date of filing: 29.10.1999
(51) Int. Cl.: A61K 9/20, A61K 9/48

(54) **Double phase time-controlled release system**

(71) Applicant: Universiteit Leiden, 2300 RA Leiden (NL)
(72) Inventor: Junginger, Hans Eugen, 2324 JN Leiden (NL); Dorkoosh, Farid Abedin, 2334 EA Leiden (NL); Verhoef, Jacobus, 3712 BW Bilthoven (NL); Rafiee-Tehrani, Rafiee, Mirdamad Ave. Teheran (IR)
(74) Representative: Ottevangers, Sietse Ulbe

(57) **Abstract**

The invention relates to a delivery system for active ingredients which comprises a swellable phase defining at least one void. In this void an active ingredient may be present. The active ingredient may be substantially enclosed by the swellable phase, optionally with a degradable layer present between the gel phase and the active ingredient. The invention further relates to a method for providing such delivery systems, a method for non-medical administration of active ingredients, as well as to the use of a swellable phase in controlled release systems.

## Description

The invention relates to a delivery system for active ingredients which comprises a swellable phase defining at least one void. In this void an active ingredient may be present. The active ingredient may be substantially enclosed by the swellable phase, optionally with a degradable layer present between the gel phase and the active ingredient. The invention further relates to a method for providing such delivery systems, a method for non-medical administration of active ingredients, as well as to the use of a swellable phase in controlled release systems.

Through recent advances in biotechnology, therapeutic compounds based on protein, peptide and nucleotide sequences structures have become readily available in reasonable quantities and prices. Disadvantages that these compounds share is their instability against acidic, alkaline and/or enzymatic degradation upon systemic uptake and additionally their poor intestinal absorption due to their high hydrophilicity. Therefore, these compounds are almost exclusively administered by parenteral injection, which has the disadvantages of painful application, risk of infections, low patient compliance and the need of trained personnel.

Usually, peroral application is the easiest and most preferred way of drug administration. For most drugs or other active ingredients of high molecular weight, however, systemic absorption is hindered by low intestinal epithelial permeability and by enzymatic degradation. An ideal delivery system for these drugs would enhance epithelial permeability and/or restrict enzymatic or other chemical activity at a pH value where these hydrophilic compounds are the most stable.

Attempts employing mucoadhesive polymers have been rather successful *in vitro* and *in vivo* (see for example: H.L. Lueßen *et al*.; Pharm. Res. 1995, 12: 1293-1298; H.L. Lueßen *et al*.; Pharm. Res. 1996, 13:1668-1672). However, the turn-over of intestinal mucus covering the epithelium led to a rapid delocalization of the polymeric delivery system (C.-M. Lehr *et al.;* Int. J. Pharm. 1991, 70:235-240). It would thus be desirable to have a system that is retained in the lumen such as at specific sites in the gut, *e.g.,* by controlled swelling over a predetermined and extended period of time. Such systems would have distinct advantages over delivery systems for active ingredients, such as drugs, known so far.

One objective of the present invention is to obtain such a delivery system; that is a delivery system that keeps the dosage form mechanically fixed at the site of action.

Another objective is to get an appropriate time-controlled drug release profile with a suitable lag time, which is necessary for absorption of certain compounds such as peptide drugs.

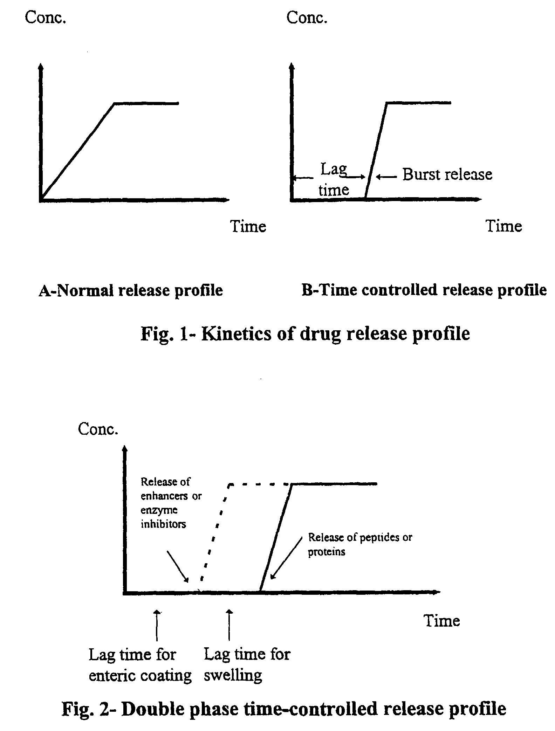

In Figure 1-A a release profile of a conventional drug release system is shown schematically. This profile is characterized by the immediate start of release (at time = 0) once the system is applied and comes into contact with the gastro-intestinal fluids.

However, in some cases, e.g. with peptide drugs, a suitable lag time is mandatory to inactivate enzyme activities and to open tight junctions. Thereafter, a burst release is necessary in which substantially the whole amount of drug can be released in a very short period of time, for example in order to achieve optimal bio-availability of the active ingredients, e.g. peptide drugs. This profile is depicted in Figure 1-B and is referred to as a "time-controlled release profile". It is another objective of the present invention to obtain a system that enables this type of release profile.

It has now been found that the above mentioned objectives can be met by a delivery system comprising a swellable phase, such as a superporous hydrogel (SPH) phase and/or a swellable superporous hydrogel composite (SPHC) phase, defining at least one void suitable to contain an active ingredient. Preferably, the dimensions of the delivery system and the chemical composition of the phases it comprises are chosen such that application of the system in a lumen results in swelling of the phase(s), which results in mechanical fixing of the system at a predetermined site in the lumen, such as of the gut.

Instead of SPH and/or SPHC other polymers can be used in the delivery system of the present invention, provided these polymers have similar swelling characteristics and have other properties that are also comparable to SPH(C).

### Brief description of the Figures

In Figure 1 two types of release profiles are depicted, showing the development in time of the concentration of active ingredients in the surroundings of the release system. Figure 1-A depicts a conventional release profile. Figure 1-B shows a time-controlled release profile, characterized by a lag time and a sudden burst release in which the concentration rises sharply.

Figure 2 shows a double phase release profile, characterized by two lag times, each followed by an increase in concentration of different active ingredients. The first lag time can for example be obtained by allowing an enteric coating around the system to dissolve or disintegrate and is followed by release of ingredients present in the polymer matrix of the system. The second lag time results from the time the system requires to swell. After swelling the second release occurs, as a result of which the concentration of active ingredients, present in the core of the system, occurs.

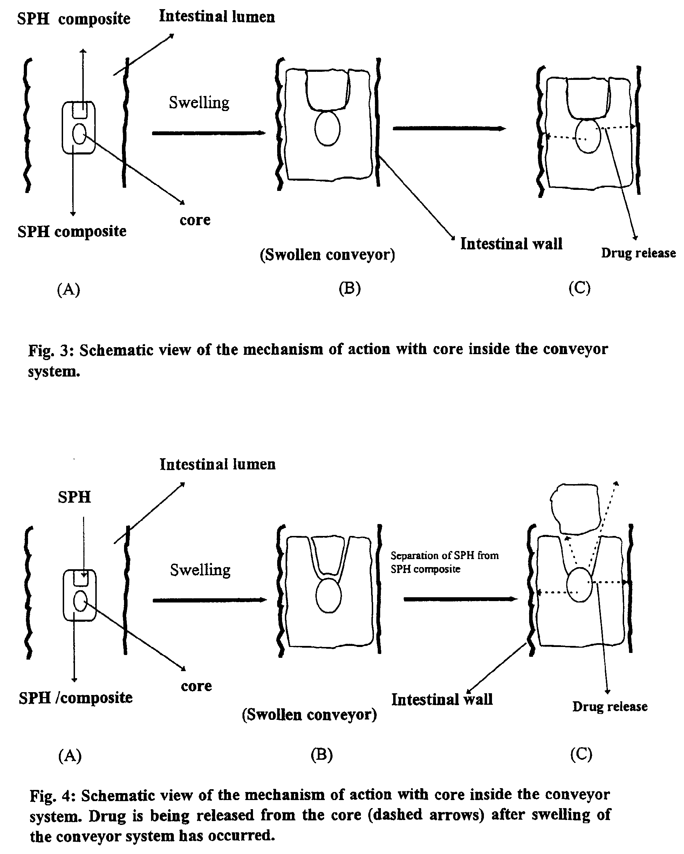

Figure 3 gives a schematic view of the mechanism of action with the core inside the body system according to the present invention. The active ingredient is released by diffusion through the swollen system.

Figure 4 shows a schematic view of the mechanism of action with the core inside the body system according to the present invention, where the body and plug have different swelling characteristics. As a result the body and plug separate upon swelling, followed by release of the active ingredient from the core.

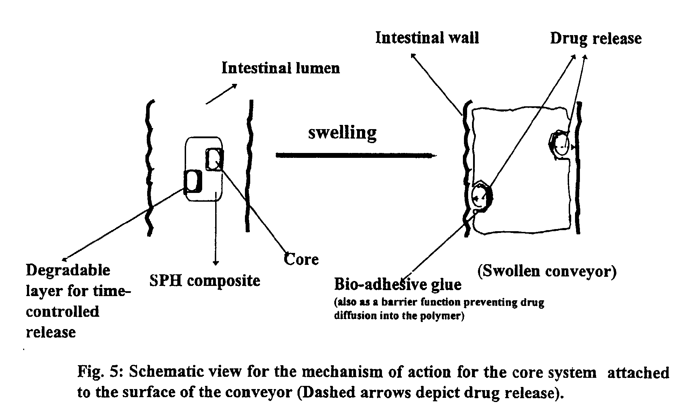

Figure 5 shows a schematic view of the mechanism of action of the core at the surface of the body system according to the present invention. The active ingredient is present in cores which are present at the surface of the body. The active ingredient is shielded from the intestinal lumen by a degradable layer. The active ingredient may be glued to the surface. This glue may also prevent diffusion of active ingredient into the matrix of the system.

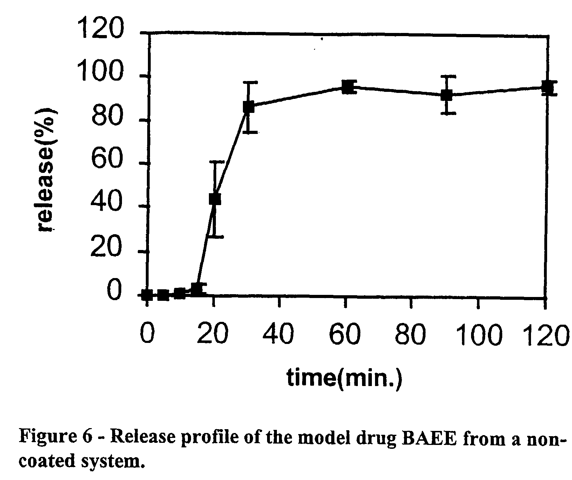

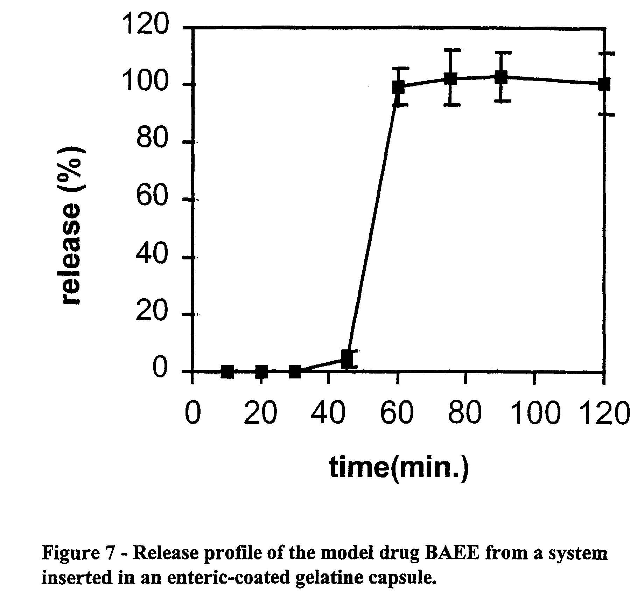

Figures 6 and 7 show release profiles as obtained in Example 1.

The void which is enclosed by said phase(s) can be referred to as the "core" of the system. It is suitable to contain an active ingredient, which can be a drug in an appropriate formulation, for example in the form of tablets, particles or small spheres. The void or core is substantially enclosed, *viz.* essentially shielded from the surroundings, by the first phase (see for example Figure 3) or substantially enclosed by a combination of said first phase and a second phase (see for example Figures 4 and 5). By substantially enclosed is meant that any active ingredient present in the void will be substantially contained therein, *i.e*. without leaking out before application. After the system is applied and as a result of the subsequent swelling process and/or of degradation processes, the active ingredient will have the opportunity to leave the system, either by diffusion through one or more of the phases which make up the system (Figure 3) or as a result of the opening of the delivery system (Figures 4 and 5). In case the void is enclosed by a combination of a first and a second phase, the first phase can be interpreted as a conveyor, having for example a cup-shaped form, and the second phase can be interpreted as the lid or plug which, together with the first phase, encloses the void.

In order to shield the active ingredient from the phases and to keep it in place, the core can be at least partially surrounded by glueing and/or barrier means (see for example Figure 5). Due to the presence of this barrier and/or glue layer, no active ingredient can diffuse into the polymer and a unidirectional release toward the intestinal wall can be obtained.

The swellable phase used in the present invention must have suitable mechanical properties. Mechanical stability is an important property for the materials used in the present invention and is expressed for example by the ultimate compression pressure (UCP), which is the pressure at which the material starts cracking. With the mechanical stability of the swellable phases used in the present invention is meant the coherent forces which keep the system together. The combination of the coherent forces and the swelling ratio of the swellable phases also influences the swelling force, *i.e.,* the force which is exerted on the lumen upon swelling.

These forces should be sufficient for the obtained delivery system to fix itself mechanically at a predetermined location in the lumen upon application and subsequent swelling.

In case the mechanical stability is too high, the system would cause problems in *in vivo* applications. A mechanical stability that is too high could cause damage to internal organs and/or would hamper the degradation progress. For this reason, the swellable phases disclosed in the prior art would not be suitable for the delivery systems of the present invention, since they are generally designed for use in long-term retention (typically more than 24 hours), such as in gastric retention.

A mechanical stability of the swellable phase that is too low, will yield a system that will not be properly fixed on the walls or will even not be fixed at all. Also a too low mechanical stability may lead to a system that falls apart during or after swelling, thereby not delivering the active material on the desired location in the lumen.

After the active ingredient is delivered, the delivery system should disintegrate, which process can be promoted by the mechanical forces exerted by the surroundings of the lumen the system is applied in. When the mechanical stability is too high, this mechanical breakdown would be hampered as well.

In general, the composition of the swellable phases used in the system of the present invention should be chosen such that a mechanical stability is obtained that falls within the above mentioned extremes. This is dependent on the lumen wherein the delivery system should be applied. By carefully choosing the amount of cross-linker and superdisintegrant or increasing the amount of foaming agent, when superporous hydrogels (SPHs) and superporous hydrogel composites (SPHCs) are employed, the mechanical stability can be fine-tuned such that their properties are appropriate for the drug delivery systems of the present invention.

Suitable UCP values for the delivery systems of the present invention range from 1 to 15 000 Pa, more preferably 10 to 500 Pa and most preferably 30 to 500 Pa. The UCP is determined by applying increasing weight to the delivery system until the point where it starts cracking. This method is described in more detail in W.S.W. Shalaby and K. Park, Pharm. Res 7 (1990), 816-823.

Materials that are suitable for the swellable phase according to the present invention comprise any material that swells upon contact with water or aqueous solutions. This water may originate for example from the fluids present in the lumen where the delivery system is applied. Suitable materials for the swellable phases of the present invention are hydrogels. Hydrogels are polymer networks which are insoluble in water, but swell to an equilibrium size in the presence of water. The water is taken up in the spaces between the polymer chains.

Porous hydrogels are hydrogels in which pores are present. Due to the presence of pores, the rate at which water is taken up, *i.e.,* the swelling rate, is considerably increased. Therefore, porous hydrogels are preferred materials for the swellable phase, used in the released system of the present invention. Porous hydrogels can be microporous, macroporous or superporous, which can be classified as having pore sizes ranging from about 0.01-0.1 µm, about 0.1-10 µm and about 10-3000 µm, respectively. From these preferred materials, superporous hydrogels are especially preferred, since they provide a high swelling rate when compared to the other porous and nonporous hydrogels. In addition, their mechanical stability can be adjusted by transforming the SPHs to SPHCs.

SPHs and SPHCs are synthesized by crosslinking polymerization of monomers in the presence of e.g. blowing agents, or other means, which create gas bubbles in a suitable solvent, such as water or organic solvents. An example of the preparation of SPHs and SPHCs can be found in WO-A-98/51408. In the synthesis of SPHs blowing agents and surfactants are required. In addition crosslinker and chemical initiator are present in the synthesis mixture. The SPH is formed by two concomitant processes: foaming and polymerization. To permanently capture the gas bubbles in the polymer network, the formation of the gel must occur when the foam is stabilized.

The properties of the SPH and the SPHC can be controlled by choosing factors such as the type and concentration of monomers, the type and concentration of initiators, the temperature and type of solvent and the amount and type of blowing agent.

The bubbles required in the synthesis may originate from any gas blowing method. The bubbles can be introduced by bubbling gas through the solution, or alternatively by adding a compound that produces gas under synthesis conditions.

SPHCs can be made from SPHs by treating the SPHs with swelling and mechanical strength controlling agents or methods. Examples of the preparation of SPHCs can be found in WO-A-98/51408 which document is incorporated herein by reference to describe the synthesis of SPHs and SPHCs.

Alternatively, SPHCs can be made by combining a monomer having at least one unsaturated bond between two carbon atoms (ethylenical unsaturation), a multiolefinic crosslinker, and particles of a fast water absorbing material, a so-called disintegrant. Subsequently, the mixture is subjected to polymerization conditions which yields the SPHC. Apart from the presence of the disintegrant, the ingredients and conditions used in the synthesis of SPHCs are further essentially similar to that used in the synthesis of SPHs.

Preferably the SPH or the SPHC used in the systems of the present invention is a polymer or copolymer of ethylenically unsaturated monomers, preferably of acrylamide (AM), N-isopropylacrylamide (NIPAM), 2-hydroxyethyl methacrylate (HEMA), 2-hydroxypropyl methacrylate (HPMA), N-vinyl pyrrolidone (VP), acrylic acid (AA), sodium acrylate, 2-acrylamido-2-methyl-1-propane-sulfonic acid (AMPS), potassium salt of 3-sulfopropyl acrylate (SPAK) and/or 2-(acryloyloxy)-ethyltrimethylammonium methyl sulfate (ATMS) whereby the SPHC is formed by crosslinking, by combining at least one (ethylenically) unsaturated monomer, a multiolefinic crosslinking agent, particles of a disintegrant and a blowing agent, such as bicarbonates or other CO₂ sources. This admixture is then subjected to polymerization and foaming conditions to polymerize and crosslink the monomer, crosslinking agent and disintegrant.

One way of modifying the breaking pressure of the system in order to make it appropriate for intestinal drug delivery, is by choosing the amount of disintegrant or superdisintegrant used in SPH and SPHC formulations. In order to obtain systems with an acceptable breaking pressure, the amount of disintegrant is preferably from 0 to less than 50 wt.% based on the mixture of monomer and disintegrant, more preferably from 0.1-10 wt.%, and most preferably 0.5-5 wt.%.

The breaking pressure can further be controlled by choosing the amount of crosslinker, which is used in the synthesis of hydrogels. Best results with respect to mechanical stability are obtained with amounts of 5-10 wt.% for SHP and 10-20 wt.% for SPHC, based on the total weight of monomer and crosslinker. Preferably these ranges are 6-9 wt.% and 13-18 wt.%, respectively, most preferably 7-8 wt.% and 15-16 wt.%, respectively.

The amount of foaming agent used with the synthesis of porous hydrogels, and in particular with the synthesis of SPHs and SPHCs is in this respect preferably 1-30, more preferably 5-20, most preferably 10-15 mole%, based on the combined number of moles of the blowing agent and the monomer.

Other important characteristics of the systems of the present invention comprise the diffusion characteristics, particle size, swelling ratio and the swelling rate.

The diffusion of compounds from the delivery system can be influenced by the pore size of porous hydrogels. The larger the pore sizes of a porous swellable material is, the easier the compounds will diffuse. Good release characteristics can be obtained with swellable materials having open porosity with pores with typical diameters of 10-1000 µm, preferably 25-200 µm, most preferably 50-100 µm.

The diameter of the system of the present invention in swollen state depends strongly on the envisaged application, in particular it depends on the size of the lumen it is applied to. The size of the non-swollen release system is determined by the diameter of the system in swollen state in combination with the swelling ratio of the swellable material. This combination should be such that mechanical fixation of the system at the desired site in the lumen can take place.

Generally the size of the swollen system will be from several mm to several cm, preferably 1-5 cm, more preferably 2-4 cm. The cross-sectional diameter of the swollen system has to be sufficient to fix the system at the desired site in the lumen.

Depending on the swell ratio, this may correspond to cross sectional diameters of the systems in non-swollen state from 0.05 to several cm, preferably 0.5-2 cm, more preferably 0.7-1.5 cm.

The swell ratio of the swellable phase is preferably from 2-200, more preferably from 20-100, and most preferably from 50-80. The swell ratio can be modified by changing the synthesis conditions, in particular the amount of crosslinker. The swell ratio is also the result of factors such as the amount of pore formation, the presence of composite substances and the concentration of monomers.

The systems of the present invention preferably display complete swelling within several minutes up to about one day after application, more preferably within 5 min to 24 h and most preferably within 25 min to 8 h.

With the systems of the present invention it is possible to deliver active ingredients at a predetermined site in the lumen the system is applied to. By carefully choosing factors such as chemical composition, size and dimensions, which in turn may influence factors such as swelling rate, swelling ratio, permeability, degradability, etc., the systems of the present invention can be designed to deliver a predetermined amount of active ingredient at a specific site in a certain lumen after a predetermined lag time, according to a certain release profile. Additionally, the release may be accompanied by the release of certain additional compounds.

It has been found that, when SPHs or SPHCs are used, by choosing the amount of disintegrant, the amount of crosslinking agent and the amount of foaming agent, as described more in detail below, systems with a suitable mechanical stability can be obtained.

In addition to mechanical stability, the diffusion characteristics of the systems should be such that a desired release profile is obtained. This can also be controlled by the above mentioned synthesis conditions, as well as other synthesis conditions. This will also be described in more detail below, as well as other important characteristics of the release systems of the present invention such as the particle size, the swelling rate and the swelling ratio.

Certain superporous hydrogels (SPHs) and superporous hydrogel compositions (SPHCs) comply with the demands of the materials used as swellable phase in the present invention and are the preferred materials. Hereinbelow the present invention will be described in detail, while referring to SPH or SPHC. However, any other swellable material, having the required properties can be used as well, and are hence suitable alternatives.

In a preferred embodiment the system comprises a first swellable phase and at least one void suitable to contain an active ingredient, wherein said first phase comprises a SPH or a SPHC, and wherein said void is either substantially enclosed by said first phase (see Figure 3) or substantially enclosed by a combination of said first phase and a second phase (see Figures 4 and 5). The delivery system thus has the possibility to encompass an active ingredient or another ingredient to be released. It has also been found that the SPH or the SPHC have swelling properties allowing the retention of the system at a specific site (drug targeting) within the intestinal tract by mechanical forces. Dependent on the types of superporous hydrogels, additionally bioadhesive interactions, such as mucoadhesive interactions can be achieved which support the mechanical fixation at a desired site in the intestinal tract.

The delivery systems of the present invention can effectively be employed in a variety of lumina in humans or animals, as well as in plants.

The delivery systems of the present invention are preferably based on superporous hydrogels (SPHs) and optionally superporous hydrogel compositions (SPHCs). SPHs and SPHCs may swell in an aqueous environment to more than 100 times of their dry volume. These polymers have been developed as gastroretentive systems (Hwang S.-J. *et al.;* Crit. Rev. Therap. Drug Carrier Sys. 1998, 15(3): 243-284). SPHs and SPHCs are hydrogels with numerous pores connected together to form open channel structures. The pores have typical dimensions of 10-3000 µm, preferably 100-500 µm, even more preferably 50-200 µm. Water can be absorbed into dried SPHs by capillary wetting of the pores. This enables fast swelling of the gels (within minutes). The swelling ratio of a gel is defined as the ratio of the volume increase due to swelling (*i.e*. the volume of the gel in swollen state minus the volume in non-swollen (dried) state) to the volume in dried state. The preferred swelling ratios of the SPHs and SPHCs used in the present invention, will depend on factors such as the envisaged application in combination with the particle size of the material in non-swollen state. Swelling should not cause damage to the lumen the system is applied to. The SPHs preferably have a swelling ratio of 10-200. The SPHCs preferably have a swelling ratio of about 10-20. The swelling occurs preferably within 1-20 min.

The SPHs or SPHCs used in the systems of the present invention are biodegradable and/or degradable into compounds that are excreted easily from the system. Mechanical breakdown of the gels or the composites is enhanced by the contraction forces of the intestinal tract and is dependent on the composition of the SPH and/or SPHC.

The delivery systems of the present invention can be applied for delivery of active ingredients in different body cavities in humans or animals or in lumina in plants. Once inserted, the polymers will swell and fill the cavity. Successively, the active ingredient is released, which will then act locally or is absorbed locally into the blood circulation or other systemic fluid compartments.

In a preferred embodiment, the delivery system of the present invention is brought into the intestinal tract and the active ingredients are intended to be absorbed into the blood circulation.

The absorption of peptide and protein drugs through the intestinal epithelium can be enhanced by: i) inhibition of luminal and brush border enzyme activity, ii) opening of tight junctions, iii) retention of the dosage form at a specific site, *e.g.,* by mucoadhesion (see for example: Davis SS, J. Pharm. Pharmacol. 1992, 44 (Suppl. 1): 186-190; Lee HJ and Amidon GL, NIDA Res. Monogr. 1995, 154:86-106; Vyas SP, Venugopalan P, Sood A and Mysore N, Pharmazie 1997, 52:339-345; Junginger HE, Verhoef JC, Pharm. Sci. Technol. Today 1998, 1:370-376).

With the system of the present invention, a so-called "double phase time-controlled release profile" can be obtained. For the absorption of hydrophilic compounds and macromolecules such as peptides and proteins initially the luminal and brush border enzymes should be inactivated and tight junctions should be opened; therefore, it is necessary to first have the release of absorption enhancers and action of enzyme inhibitors effected and subsequently, after a specific period of time in which the enzymes are inactivated and tight junctions are opened, to have the burst release of active compound started. The double phase time-controlled release profile which can be obtained by the present invention is illustrated in Figure 2. Because peptide and protein drugs will be degraded in the stomach fluids, the dosage form has to have a conventional enteric coating. After swallowing such an enteric coated delivery system (preferably incorporated in a suitable gelatin capsule), there will be no release of active compounds as long as the delivery system remains in the stomach (lag time for enteric coating). After having passed the pylorus and being surrounded by intestinal liquids with a pH between 5.5 and 7.5, the enteric coating will dissolve.

The swellable phase which constitutes the system of the present invention, may apart from a suitable swellable material, such as SPH and/or SPHC and optionally the material of the degradable layer, comprise other ingredients. The phases may further comprise water or aqueous solutions, which will lead to swelling of the SPH or SPHC. Other (mixtures of) additional ingredients that may be present to improve the characteristics of the system of the present invention are absorption enhancers (also referred to as penetration enhancers), enzyme inhibitors, gut motility control substances, substances which influence the adhesion to mucosa, disintegrants, binders, lubricants and/or mixtures thereof.

The second phase may comprise, like the first phase, a swellable phase and preferably a SPH or a SPHC. In an alternative embodiment it is also possible to use a layer of a suitable material to fulfill the enclosing function. This embodiment is for example characterized by a conveyor having one or more holes or pores on its outer surface which holes constitute the voids or cores. These holes have a typical dimension of 1 to 10 mm. These voids can contain the active ingredient and are closed by a degradable layer. The layer will upon application at a certain point in time disintegrate, e.g. by melting or dissolving, after which the active ingredient can be released. Preferably such a degradable layer comprises hydroxypropyl methylcellulose (HPMC), methylcellulose (MC), cocoa butter, hydrogenated castor oil or mixtures thereof, short or medium chain triglycerides (Miglyole^{™}), polyglycerol fatty acid derivatives (PGEF).

In order to increase epithelial permeability, all compounds allowed to be used as absorption enhancers can be integrated in the SPH and/or SPHC. Examples of such compounds are edetate (EDTA), polyacrylates (Carbomer), chitosans and their derivatives, anionic surfactants such as sodium lauryl sulfate, nonionic surfactants such as polyoxyethylene ethers and esters, fatty acids such as sodium caprate, trihydroxy bile salts such as taurocholate, dihydroxy bile salts such as taurodeoxycholate, acyl carnitines such as palmitoyl carnitine and salicylates such as sodium salicylate.

In order to reduce enzymatic activity all known classes of protease inhibitors and other enzyme inhibiting substances can be added to the SPH and/or SPHC comprising phase. Enzyme inhibitors improve the absorption of macromolecular drugs. During the synthesis process of the SPH and/or SPHC, substances with enzyme inhibiting activity can be covalently bound to the SPH and/or SPHC. Compounds which increase epithelial permeability and reduce enzymatic activity simultaneously, can also be added. Suitable enzyme inhibitors are aprotinin, cystatin, soybean trypsin inhibitor, leupeptin, bestatin and small inhibitors based on boron compounds, such as α-aminoboronic acid derivatives.

Another application possibility is the use of ketoconazol and levamisol as enzyme inhibitors for cytochromes such as cytochrome P450 and especially for cytochrome P3A4 and for other drug transporters such as P-glycoproteins within the gut wall to overcome multidrug resistance of drugs especially anti-cancer drugs.

Further optional ingredients are compounds that can control the intestinal motility, *viz*. either decrease the motility for prolonged retention time of the dosage form at the site of action or increase the motility for easier excretion of the dosage form. These compounds can influence the transition of the system through specific parts of the intestine. Examples of such compounds are loperamide HCl, papaverine HCl, opiate receptor stimulators and acetylcholine antagonists.

Also, it is possible to increase the adhesion and/or covalent binding of the system to the mucosa, by using compounds such as slightly cross-linked poly(acrylic acid), which is commercially available as polycarbophil and Carbopol™ which also can be formed from suitable monomers during the synthesis of SPH/SPHC. Other mucoadhesive polymers are chitosan derivatives, carboxymethylcellulose and other polymers.

Further additional ingredients comprise disintegrants and superdisintegrants, binders and lubricants.

The systems of the present invention can be used for various pharmacologically active substances, including drugs, vaccines and their components (*e.g*., isolated antigens or parts thereof), pharmacologically active substances such as peptides, proteins and polysaccharides, internal wound dressings, local therapeutic drugs for the GI tract, monoclonal antibodies, oligonucleotides, and genes. For application in plants the systems may comprise as active ingredients insecticides, fungicides, germicides, nutrients, *etc.*

It is possible to achieve desirable kinetics for the release profile by modifying the formulations and swelling ratio of the system.

The systems of the present invention can be used *inter alia* for the delivery of either natural or synthetic modifications of the following pharmacologically active compounds: gonadotropins (FSH, LH), thyrotropin (TSH), vasopressin, adrenal hormones including corticosteroids, pancreatic hormones such as insulin and glucagon, thyroid hormones such as thyroglobulin, sex steroid hormones such as estradiol, progesterone, testosterone, and nandrolone, calcitonins, encephalins, thymic humoral factor (THF), growth hormone releasing hormone (GHRH), luteinising hormone releasing hormone (LHRH) and its analogues (Buserelin), calcitonin gene related peptide (CGRP), vaccines (such as AIDS vaccines, hepatitis B vaccine), cholecystykinin, atrial natriuretic peptide.

Other drugs that can be applied by the systems of the present invention are: antibiotics and antimicrobial agents like penicillin derivatives, different generations of cephalosporines, sulfonamides, dactinomycin, fluorouracil, tetracyclines, pentamidine, erythromycin, and nitrofurazone; antimetabolite agents such as methotrexate, thiotepa; diagnostic drugs such as azuresin, betazole hydrochloride; vitamins such as vitamin K, cyanocobalamin; enzymes such as penicillinase; local anesthetics such as benzocaine, chloroprocaine hydrochloride; sedatives and hypnotics such as barbital, amobarbital, meprobamate; antiseptics such as chlorhexidine, benzalkonium chloride; psychopharmacologic agents such as antiepileptics (like primidone), tranquillizing agents (like chlorprothixene), antidepressant agents (like doxepine hydrochloride); analgesics and antipyretics such as codeine, nalorphine; nonsteroidal antiinflammatory drugs such as acetylsalicylic acid; antihistaminic agents such as dexchlorpheniramine, diphenhydramine, terphenadine; central nervous system stimulants such as caffeine, theophylline; antiemetics such as cyclizine hydrochloride; antiallergics such as clemastine fumarate; antitussive agents such as dextromethorphan, noscapine; antiasthmatic agents such as isoproterenol; hematopoietics such as detriferron, iron dextran; anticoagulants such as anisindione, heparin; antihypertensive agents such as trimethaphan camsylate, mebutamate, methyldopa, nifedipine; vasodilators such as papaverine; coronary drugs such as nitroglycerine; cardiotonics such as digoxin, deslanoside; antiarrhythmic drugs such as quinidine; blood lipid lowering agents such as clofibrate, sitosterols; vasoconstrictors such as phenylephrine, naphazoline, oxymetazoline; cholinergic agents such as pilocarpine, edrophonium chloride; anticholinesterases such as physostigmine, demecarium bromide; adrenergic blocking agents such as phentolamine mesylate, reserpine; antimuscarinic drugs such as atropine, glycopyrrolate; neuromuscular blocking agents such as succinylchloline chloride; antiparkinson drugs such as biperiden; diuretics such as furosemide, hydrochlorothiazide; uterine drugs such as ergonovine maleate, oxytocin.

When applied, the release system of the present invention takes the core to the site of action. By its fast swelling properties it can attach to the wall of the lumen it is applied to and through mechanical interaction it can keep the dosage form at the site of action. The general concept of this system is suited for a variety of applications.

For all embodiments of the present invention absorption enhancers, enzyme inhibitors and other optional additives can be added at the beginning or during the synthesis process of the phases. It is also possible to impregnate the systems after synthesis with a solution comprising the additives. If appropriate, for example in case these compounds are incompatible with synthesis conditions, these compounds can also be added after the synthesis process of the conveyor systems. It can be especially desirable that most of any fluid if present in the respective section of the lumen is taken up by the conveyor system and that the enzymes dissolved or suspended in these fluids are taken up as well and then deactivated inside the conveyor systems. Brush border enzymes can be deactivated by direct interaction with enzyme inhibitors (covalently) attached to the conveyor or by the release of suitable amounts of enzyme inhibitors from the conveyor system to the lumen surface.

Also the efflux of water into the lumen, *e.g.* from gut tissue, can generally be used for the complete swelling of the conveyor system, in order to increase the permeability of the membrane surrounding of the respective lumen and to enhance (peptide) drug absorption.

Polymers that can be used for time-controlled release comprise hydroxypropyl methylcellulose (HPMC), methylcellulose (MC), and other derivatives of cellulose. In addition to polymers, hydrogenated castor oil and other lipids melting at 37°C, such as cocoa fat can be used. Release can also be modified by the thickness of these layers.

The systems of the present invention can be used in a wide variety of applications, such as targeting drugs to specific sites in intestinal, vaginal, nasal, oral, rectal and ear cavities, the colon and the lung. Other possible applications are in surgery for the delivery of drugs like antibiotics to surgical wounds and the mechanical fixation of implants.

The invention is further characterized by a method for the non-medical administration of an active ingredient to a predetermined site in a lumen. This method comprises incorporating the active ingredient in a void present in a swellable body and inserting said body in said lumen under such conditions that swelling occurs, which swelling results in said ingredient being dispensed essentially at said predetermined site. Examples of compounds that may be used in this method as active ingredients comprise vitamins, nutriceuticals and antiseptic agents.

The conditions at which swelling occurs are essentially as described above.

Two embodiments of the systems of the present invention are now further described in more detail and with reference to the drawings: a system in which the core is inside the body, and a system in which the core is attached to the surface of the body.

### 1. Core inside the body

In this embodiment of the drug delivery system of the present invention, the first phase is a body in a cup-shaped form and the second phase is a plug. The system has a coating that is formed by an enteric coated capsule, which coating is chosen such that it dissolves in the intestinal lumen. The first and second phases are chosen such that, upon contact with intestinal fluids, swelling of the system occurs. By choosing the composition of the phases, the swelling results in the system being substantially fixed at a suitable location in said lumen. Subsequently the additives, if present, are released by diffusion through the pores of the gel material.

This system is schematically depicted in Figure 3, which shows the core void, including the active ingredient, which is incorporated inside of the conveyor system of a suitable shape, made from SPH or SPHC. The conveyor system, or 'body', preferably has a cylindrical shape with a cylindrical hole in which the drug-loaded core is inserted, as depicted in Figure 3-A. The conveyor system is closed by a plug of the same SPH or SPHC material. The SPH and/or SPHC conveyor system is inserted in an enteric coated gelatin capsule. After application, dissolving of the enteric coating in the intestinal fluids will occur, as a result of which the system swells and remains fixed at the desired place of drug absorption in the intestinal tract (Figure 3-B). Subsequently the necessary amounts of absorption enhancers and enzyme inhibitors can be released, if this is required. Thereafter, the drug is released (as depicted in Figure 3-C). This system has typical dimensions of 0.5-5 cm, preferably 1-3 cm, before application, *viz.* in non-swollen form. The size will depend on the swelling ratio of the materials used in combination with the envisaged application.

In Figure 3 a schematic view of the mechanism of action with core inside the body system is given. Drug is being released from the core (dashed arrows) after swelling of the conveyor system has occurred.

This embodiment can be made from a body and a plug comprising the same SPH or SPHC. The materials are in this case are chosen such that the active ingredient, present in the core void, is released by diffusion, essentially after the system is fixed.

In a special case of this system the body and the plug comprise different superporous hydrogels (SPHs) and/or superporous hydrogel composites (SPHCs). The composition of the phases is chosen such that the body and the plug have different swelling ratios, which difference in swelling ratios is such that it results in separation of plug and body upon swelling. Preferably the swelling ratio of the SPHC is about 10-20. The swelling ratio of the SPH is usually around 100. The separation of plug and conveyor occurs preferably after the system has been substantially fixed, although during its way to the desired location, some release of active ingredient may occur. Once the body swells and is fixed the body and plug separate and the active ingredient is burst-released. Preferably the body is formed of SPHC and the plug is formed of SPH in this specific embodiment.

Figure 4 illustrates this system, in which the body is formed from an SPHC comprising phase and is closed with a plug made of SPH (Figure 4-A). Due to the differences between SPH and SPHC, SPH swells more quickly. The use of SPHC as a conveyor system guarantees resistance to peristaltic movement of the intestine, keeping the system in place through mechanical interactions (Figure 4-B). The SPH plug will swell more rapidly and separate apart, thus allowing a burst release of the active compound incorporated in the core directly into the lumen (Figure 4-C).

The active ingredient used in the core-inside-the-body system can be in the form of microparticles having typical dimensions of 100-5 000 µm. These microparticles can be made using a suitable binder such as polyglycerolesters of fatty acids (PGEFs), a mixture of tetraglycerol pentastearate (TGPS, HLB 2.6) and tetraglycerol monostearate (TGMS, HLB 8.4), polyacrylate C934P (Carbomer), polyethylene glycol having a molecular weight of between 1 000 to 10 000 Da; more preferably 2 000 to 8 000 Da; most preferably 4 000 to 6 000 Da.

Additionally, the active ingredient in the microparticles can comprise a superdisintegrant or disintegrant, such as Explotab™, crosslinked Kolidon™, sodium carboxymethylcellulose (NaCMC) and other starch derivatives, and/or other osmotic agents in the form of particles (typical size 10-2000 µm) with suitable thickness to allow for an exploding system for burst release of the active ingredients after sufficient water has been able to penetrate into the core.

Typically, the formulation of an active ingredient proceeds as follows. PEG (molecular weight 4 000-10 000 Da, 30-300 mg) is molten at 60°C using a heater. Then BAEE (N-α-benzoyl-L-arginine ethylester, 1-30 mg) is added to the molten PEG and dispersed thoroughly while the dispersion is cooled down slowly. Thereafter, the formed mass is crushed in a mortar and sieved by the use of sieve mesh size 400 µm, and the microparticles less than 400 pm are incorporated inside of SPH and/or SPHC as a core.

Another possible formulation to prepare microparticles of BAEE is by using the following composition:

| | |
|---|---|
| BAEE | 1-30 mg |
| C934P | 5-40 mg |
| TGMS | 10-40 mg |
| TGPS | 30-120 mg |

Initially, TGPS and TGMS are molten together at 70°C and then C934P is dispersed in molten PGEFs. Thereafter BAEE is added and dispersed completely while the whole dispersion is cooled down gradually. The cooled mass is crushed in a mortar and sieved mesh size 400 µm. Microparticles smaller than 400 µm and especially in the range of 200-400 µm are used as a core.

After incorporation of microparticles inside of SPH and/or SPHC, the system is closed with a small piece of SPH.

Then the whole system is inserted into a conventional capsule, such as a gelatin or HPMC capsule size 000.

For delivery purposes into the intestine, the developed dosage forms should be prevented from opening in the acidic pH of stomach. Therefore, the capsules are enteric-coated, as mentioned above.

For enteric-coating of capsules, for instance the so-called pan coating method can be used which may be carried out with the ERWEKA™ apparatus (Heusenstamm, Germany). Typically this apparatus is operated at 25 rpm and a spraying rate of 1 ml coating solution every 30 sec to 1 min, the time being related to the amount of heat which is applied for drying of the coating solution. Coating of gelatin capsules requires special care, because gelatin absorbs aqueous solutions very easily and as a result the coating will be easily deformed. Therefore gelatin capsules are advantageously initially coated by using a solution of polyvinyl pyrrolidone in isopropanol (preferably 0.5 to 5%; more preferably 1 to 4%; most preferably 2 to 3%) as a protective layer and then a solution of Eudragit S100 (preferably 0.5 to 20%; more preferably 1 to 10%; most preferably 2 to 6%) is used as an enteric-coat layer. These two layers will prevent from opening of the dosage form in the acidic pH of stomach, and when the dosage form has reached the basic pH of intestine, the release of drug will be started. By changing the concentration of coating layers and also the amount of coating solutions which can be applied on gelatin capsules, it is possible to target the dosage form to specific sites in the intestine and to get drug release at a desirable site of the action.

After synthesis, the polymers are purified, for example by dialysis using a dialysis membrane having a typical molecular weight cut-off of 6000 Da, for a period sufficiently long to remove unreacted monomers, *e.g*. for 5 days. The swollen polymers can be dried by one of the two following procedures:
1) At ambient temperature in a clean environment. The drying time is dependent on the average pore size of the system and is approximately one week.
2) Under reduced pressure at 50 to 80°C. Drying time is approximately one day.

During both drying procedures the polymers shrink. Dependent on a predetermined shrinking ratio, the void of the hydrated polymers can be adapted in such a way that the dried polymers fit in gelatin or hydroxypropyl methyl cellulose (HPMC) capsules of different size, preferably of a size from 0 to 000. Mechanical modification of the polymers can be done both in the swollen and dried state. This conveyor system can be enteric coated to obtain a delayed swelling (*e.g*., at a specific site of the intestinal tract). The capsule material can be coated with appropriate polymers such as polymethacrylate derivatives, such as Eudragits®, HPMC phthalates, and others.

### 2. Core at the surface of the body

This embodiment of a delivery system of the present invention is characterized by having a body as the first phase in which body holes are made at the surface, which holes serve as the void to contain the active ingredient. The second phase can be coated by a protective degradable layer which shields the active ingredient from its environment. The entire system has a coating that is formed by an enteric coated capsule. This coating is able to dissolve upon contact with the fluids in the lumen. The first phase is chosen such that, upon contact with the fluids, swelling of the system occurs and that this swelling results in the system being substantially fixed at a suitable location in said lumen. Subsequently the additives, if present, can be released by diffusion, essentially after the system is fixed, although some release prior to fixation can occur. The composition of the degradable layer is chosen such that, essentially after the system has been substantially fixed, the degradable layer at least partially disintegrates, whereby the active ingredient which is present in at least one of said voids, is released.

In Figure 5 a schematic view of the mechanism of action for the core system attached to the surface of the body is given. In Figure 5 dashed arrows depict drug release.

In this system SPH or SPHC is used as a conveyor, *viz.* the body of the system. SPHC is mechanically more stable than SPH, thus after swelling it can be fixed mechanically to the wall of the lumen, as depicted in Figure 5, and will keep the dosage form at the site of action. Therefore, the core which includes the active ingredient, is in the nearest possible position of the gut wall and the drug can be taken up efficiently by the intestinal epithelium. If SPH is applied instead of SPHC in this system, because of its lower mechanical stability, it will break up more easily by the peristaltic pressure of the intestine and with such a system the fixation time (residence time) in the intestinal lumen, at an appropriate site can be adjusted and modified. For strong fixation SPHC is preferred in this system.

The core containing drug is sealed at the outside surface of the conveyor by a degradable layer to allow drug release only after complete swelling of the SPH or SPHC.

Dependent on the synthesis processes (amount of pore formation, crosslinking and use of composite substances) and the ratio of monomers and crosslinkers, conveyor systems can be achieved which swell within 10 min up to 24 hrs with an increased swollen volume in the range 10-100 times of the volume of the dry SPH or SPHC.

SPHC used for the core-at-the-surface system can be synthesized using methods based on what is described in WO-A-98/51408. The synthesized polymer is subsequently purified, for example by dialysis using a dialysis membrane having a typical molecular weight cut-off of 6000 Da, for a period sufficiently long (e.g. for 5 days) to remove unreacted monomers, which may be entrapped in the pores of the polymer. As far as polymers are swollen at this stage, it is easy to make small holes of appropriate size of typically 1 to 10 mm on the sides of the conveyor system (see Figure 5). Then the polymers can be dried by one of the following methods:
1) At ambient temperature in a clean environment. The drying time is dependent on the average pore size of the system and is approximately one week.
2) Under reduced pressure at 50 to 80°C. Drying time is approximately one day.

During both drying procedures the polymers shrink. Dependent on a predetermined shrinking ratio, the volume of the hydrated polymers can be adapted in such a way that the dried polymers fit in gelatin or HPMC capsules of different size, preferably of a size from 0 to 000. Mechanical modification of the polymers can be done both in the swollen and dried state.

The holes on the outer surface of the body can be made by any conventional means, *e.g*. by using lasers, blades, knives, drills or a combination of those.

After preparing the conveyor system, the core is filled. For this system the active ingredient, for example in the form of small tablets, is fitted in the small holes which have been previously formed at the surface of the polymer. These tablets can be attached to polymer by bio-adhesive glue that also has a barrier function. Due to the presence of this barrier and/or glue layer, no active ingredient can diffuse into the polymer and a unidirectional release toward the intestinal wall can be obtained. It was found that an aqueous Arabic gum solution with a concentration of 10 to 60 wt.%, more preferably 20 to 50 wt.%, most preferably 30 to 40 wt.% is very suitable for attaching tablets to the SPH and/or SPHC. Other suitable glueing/barrier compounds are > 40 wt.% aqueous solutions of synthetic derivatives of starch, such as methylcellulose, carboxymethylcellulose, or sodium alginate.

The active ingredient can also be present in the form of microparticles. Typically, the active ingredient for use in the system having the core on the surface is made by starting from a mixture of the following composition:

| | |
|---|---|
| BAEE | 1 - 30 mg |
| PEG (4 000 to 10 000 Da) | 30 - 300 mg |
| Mg stearate | 0.1 - 5 mg |

Additionally, the outer surface of the core can be coated with appropriate excipients such as swellable matrices that let the drug be released after a desirable lag time, so that a time-controlled release profile will be achieved.

Another typical composition of the active ingredient for use in the system is:

| | |
|---|---|
| BAEE | 1 - 30 mg |
| Ac-di-sol™ | 15 - 45 mg |
| Lactose | 50 - 250 mg |
| Mg stearate | 0.1 - 5 mg |

Ac-di-sol™ is cross-linked sodium carboxymethylcellulose. BAEE is mixed with lactose as filler. Thereafter, Ac-di-sol is added to the mixture and blended completely therewith. Just before making the tablets, Mg stearate is mixed with powders for approximately 5 min. Then the tablets with a hardness of 2 to 6 (more preferably 3 to 4) kPa are prepared in single punch tableting machine. The size of tablets are 2-3 mm in thickness and 3-4 mm in diameter. The method of attaching tablets to the polymer is the same as in the above mentioned procedure.

The prepared systems which have been inserted in gelatin capsules can be enteric-coated as described above.

The invention will now be illustrated by the following examples.

### Example 1. Core inside the body

SPH was synthesized by adding subsequently to a test tube solutions of the following compounds in Milli-Q water (concentrations in wt.%). The pH of the monomer solutions was adjusted to 5.5 with 10 M NaOH.

300 µl acrylamide 50%, 200 µl acrylic acid 50%, 70 µl N,N'-methylene bisacrylamide 2.5%, 300 µl Milli-Q water, 30 µl Pluronic F127 10%, 25 µl ammonium persulfate 20% and 25 µl N,N,N',N'-tetramethylethylenediamine 20%. After mixing, 100 mg sodium bicarbonate was added.

For the synthesis of SPHC 1.2 ml acrylamide 50%, 0.9 ml potassium salt of 3-sulfopropyl acrylate 50%, 0.3 ml N,N'-methylene bisacrylamide 2.5%, 90 µl Pluronic F127 10%, 30 µl acrylic acid 50%, 45 µl ammonium persulfate 20%, 120 mg crosscarmellose sodium (Ac-Di-Sol™), 45 µl N,N,N',N'-tetramethyl ethylenediamine 20% and 120 mg sodium bicarbonate were added one by one to a test tube. After addition of each ingredient the tube was vigorously shaken.

SPHC, which is mechanically more stable than SPH, was used as the body of the conveyor system and SPH, which has a much higher swelling ratio than SPHC, was used as a cap for the conveyor system. After synthesising SPHC and dialysing the polymer by the use of dialysis membrane (cut-off 6 000 Da) in order to get rid of monomers, the polymers are in the swollen state. At this stage a hole was made inside of the SPHC phase by use of a borer, to provide a void suitable to contain an active ingredient. Thereafter, the polymers were dried under reduced pressure at 60°C for one day.

During drying procedure the polymers shrank such that they could be fitted in gelatin capsules (size 000). Then, the active ingredient was incorporated inside the body of the conveyor system and this system was capped by a piece of SPH.

The active ingredient used in this example was a mixture of N-a-benzoyl-L-arginine ethylester (BAEE, 10 mg) mixed with 50 mg PEG, which is prepared as follows. Initially, BAEE is dispersed in molten PEG. After cooling, the mixture is crushed in a mortar and sieved through sieve mesh size 400 µm. The obtained microparticles were in the range between 200-400 µm and were subsequently incorporated in the SPHC conveyor as a core.

### Example 2. Core at the surface of the body

SPHC material was prepared as in Example 1. Two holes were made at opposite sides at the surface of the SPHC phase during the swollen state. The core comprising the active ingredient (corresponding to 5 mg BAEE in each hole) was attached to the surface of the polymer by one drop of 40% aqueous solution of arabic gum.

The active ingredient for use in this system had the following composition:

| | |
|---|---|
| BAEE | 10 mg |
| PEG (4 000 to 10 000 Da) | 100 mg |
| Mg stearate | 1 mg |

The preparation of the active ingredient was carried out as in Example 1, only the Mg stearate was mixed with the microparticles just before making the tablets. Then tablets with a hardness of 3.5 kPa are prepared in single punch tabletting machine. The size of tablets are 2.5 mm in thickness and 3.5 mm in diameter.

An important aspect is the attachment of these small tablets to the polymer. When the polymer swells, the size of holes in which the tablets are placed will be enlarged, and the tablets will come out of their place before they attach to the intestinal wall by the swelling of the polymer. Therefore, a bio-adhesive glue is needed to attach the tablets to the polymer in such a way that when the polymer swells, the tablets will be kept inside of the holes, up to the time the polymer swells and keeps the dosage form at the site of action. This bio-adhesive glue also functions as a barrier, and simultaneously prevents from diffusion of drug into the polymer.

After preparation of the system, it is placed in gelatin capsule size 000 and the capsules are enteric-coated by the method which has been mentioned previously

### Example 3. Core at the surface of the body

Example 2 was repeated using a mixture of the active ingredient of the following composition:

| | |
|---|---|
| BAEE | 10 mg |
| Ac-di-sol™ | 20 mg |
| Lactose | 80 mg |
| Mg stearate | 1 mg |

Initially, BAEE is blended with lactose and secondly with Ac-di-sol. Then, just before making the tablets, the microparticles are mixed with Mg stearate and the tablets with a hardness of 3.5 kPa are prepared in a conventional single punch tabletting machine. The size of tablets are 2.5 mm in thickness and 3.5 mm in diameter.

After preparation of the system, it is placed in gelatin capsule size 000 and the capsules are enteric-coated by the method which has been mentioned previously.

### Release patterns

The dissolution of a coated and a non-coated delivery system according to the present invention was investigated by using the standard USP XXIII dissolution paddle apparatus. The paddle speed was 100 rpm and the dissolution medium was phosphate buffer solution (PBS) pH 7.2 at 37±1°C. PBS was prepared according to the method of USP XXIII. Three replicates for each sample were tested at specific interval times and their mean percent release was calculated.

Two release patterns of example 1, showing the release profiles of BAEE from the system with and without a gelatin enteric coating (capsule) are depicted in Figures 6 and 7, respectively.

Figure 6 shows a release profile of BAEE from the system when it is not inserted in gelatin capsule. Data are expressed as mean ± standard deviation (SD, n=6). (Standard USP XXIII dissolution apparatus is used with Phosphate Buffer Solution (PBS) pH = 7.2 as a dissolution medium. Temperature was 37±1°C and paddle speed was 100 rpm).

Figure 7 shows a release profile of BAEE from the system when it is inserted in gelatin capsule and enteric-coated. Data are expressed as mean ±SD (n=6). (Standard USP XXIII dissolution apparatus is used with PBS pH = 7.2 as a dissolution medium. Temperature was 37±1°C and paddle speed was 100 rpm).

As observed from Figure 6, when the core comprises BAEE as a model drug incorporated in the polymer, after a lag time of approximately 10 to 20 min, a burst release is achieved. Within a short period of time the whole amount of drug is released and a desirable release profile is obtained. Figure 7 illustrates that when the system is inserted in a gelatin capsule which is enteric-coated, the lag time will be increased. A lag-time for burst release of the active ingredients of 10-60 min, preferably 20-30 min is most favorable to achieve enzyme deactivation at the fixation area and to achieve opening of the tight junctions of the intestinal epithelium before the active ingredients are released. This is especially important when drugs are used that are sensitive to enzymatic degradation, such as peptide drugs. Initially the coating layer and gelatin capsule should be dissolved an then the release from the system will be started. Because these processes have their own lag time prior to the swelling of the system, these results are in accordance a "double phase time-controlled release profile".

## Claims

1. Delivery system comprising a swellable phase defining at least one void suitable to contain an active ingredient.

2. Delivery system according to claim 1 in which a first swellable phase comprises a superporous hydrogel (SPH) or a superporous hydrogel composite (SPHC), and wherein said void is either substantially enclosed by said first phase or substantially enclosed by a combination of said first phase and a second phase.

3. Delivery system according to claim 2 wherein the SPH or the SPHC is comprised of a polymer or copolymer of acrylamide (AM), N-isopropylacrylamide (NIPAM), 2-hydroxyethyl methacrylate (HEMA), 2-hydroxypropyl methacrylate (HPMA), N-vinyl pyrrolidone (VP), acrylic acid (AA), sodium acrylate, 2-acrylamido-2-methyl-1-propanesulfonic acid (AMPS), potassium salt of 3-sulfopropyl acrylate (SPAK) and/or 2-(acryloyloxy)ethyltrimethylammonium methyl sulfate (ATMS) whereby the SPHC is formed by combining at least one unsaturated monomer, a multiolefinic crosslinking agent, particles of a disintegerant and a blowing agent.

4. Delivery system according to claim 2 or 3 in which said second phase comprises a SPH, a SPHC or a degradable layer.

5. Delivery system according to claim 4 in which said degradable layer is selected from the group consisting of hydroxypropyl methylcellulose (HPMC), methylcellulose (MC), cocoa butter, hydrogenated castor oil, short or medium chain triglycerides, polyglycerol fatty acid derivatives and composites thereof.

6. Delivery system according to any of the previous claims in which said active ingredient is at least partly surrounded by glueing and/or barrier means.

7. Delivery system according to any of the previous claims in which said void encompasses a pharmaceutical composition as the active ingredient.

8. Delivery system according to any of the previous claims in which said swellable phase, said first and/or said second phase contains one or more additives chosen from the group consisting of absorption enhancers, enzyme inhibitors, gut-motility control substances, substances which influence the adhesion to mucosa, disintegrants, binders, lubricants and mixtures thereof.

9. Delivery system according to any of the previous claims which system is coated, preferably enteric coated.

10. Delivery system according to anyone of the preceding claims 3-9 in which said first phase has a cup-shaped form and said second phase has the form of a plug, leaving a void in the first phase, said phases being chosen such that, upon swelling additives, present in the void, are released by diffusion.

11. Delivery system according to claim 10 in which the body and the plug comprise different superporous hydrogels (SPHs) and/or superporous hydrogel composites (SPHCs), the composition of said phases further being chosen such that said body and said plug have different swelling ratios such that said plug is separated from the body upon swelling and the active ingredient, present in said void, is burst-released.

12. Delivery system according to claim 11 in which the body comprises SPHC and the plug comprises SPH.

13. Delivery system according to anyone of the preceding claims 2-10 in which said first phase is a body having at least one void in the form of a hole on the outer surface of said body and said second phase is a degradable layer, said phases being chosen such that, upon swelling additives, present in the void, are released by diffusion.

14. Delivery system according to any of the previous claims in which said swellable phase has a swelling ratio of 1:10 to 1:200.

15. Delivery system according to any of the previous claims for the targeting of drugs to specific sites in the ileum, the colon, the lung; or the oral, rectal, nasal, ear or vaginal cavity.

16. Use of a swellable phase for the delivery of nutrients and/or drugs over an extended period of time in a specific cavity of a human or animal body or in a cavity of a plant.

17. Method for providing a drug delivery system comprising the steps of a) forming a body of SPH and/or SPHC phase comprising a hole, b) filling said hole with a pharmaceutical composition and optionally with glueing and/or barrier means, c) closing said hole with a plug comprising SPH and/or SPHC phase, and d) coating the product of the previous step with a suitable coating, preferably an enteric coating.

18. Method for the non-medical administration of an active ingredient to a predetermined site in a lumen comprising incorporating said ingredient in a void present in a swellable body and inserting said body in said lumen under such conditions that swelling occurs, which swelling results in said ingredient being dispensed essentially at said predetermined site.

19. Use of a combination of SPH and SPHC in a controlled release system.

20. Use of a combination of SPH and SPHC for the mechanical fixation of implants.
